# EUROPÄISCHE PATENTSCHRIFT

(11) **EP 0 955 495 B1**
(45) Veröffentlichungstag und Bekanntmachung des Hinweises auf die Patenterteilung: **25.06.2003**
(21) Anmeldenummer: 99108189.4
(22) Anmeldetag: 27.04.1999
(51) Int. Cl.: F16L 37/42

(54) **Steckkupplungsventil für zentrale Gasversorgungsanlagen**
Valved plug-in coupling for gas supply systems
Raccord enfichable à soupape pour systèmes d'alimentation en gaz

(30) Priorität: 04.05.1998 DE 19819358; 09.07.1998 DE 19830690
(43) Veröffentlichungstag der Anmeldung: 10.11.1999
(73) Patentinhaber: Heraeus Med GmbH, D-63450 Hanau (DE)
(72) Erfinder: Knoop, Thomas, 42855 Remscheid (DE); Scholz, Bernhard, 63303 Dreieich (DE); Grünewald, Thomas, 64747 Breuberg (DE)
(74) Vertreter: Thoenes, Dieter, Dr.

(56) Entgegenhaltungen:
- DE-A- 2 705 319
- DE-A- 3 133 772
- US-A- 5 630 570

## Beschreibung

Die Erfindung betrifft ein Steckkupplungsventil für zentrale - insbesondere für medizinische - Gasversorgungsanlagen mit zwei jeweils paarweise gegenüberliegend auf einer Längsachse eines zugehörigen Ventilgehäuses versetzt angeordneten federbelasteten Verriegelungselementen zur Arretierung eines durch eine Frontöffnung eines federbelasteten Druckrings (als Schiebehülse) entlang der Längsachse in seine Arbeitsstellung einsetzbaren Anschluss-Steckers mit hülsenförmiger Spitze zur Gasaufnahme, wobei einerseits das erste Paar von Verriegelungselementen eine Arretierfläche in Form eines die Spitze des Anschluss-Steckers umlaufenden Bundes blockiert, andererseits bei Entnahme des Anschluss-Steckers zwei nacheinander folgende Schubbewegungen des Druckrings mit dazwischen liegender Parkposition vorgesehen sind, wobei in der Parkposition das erste Paar von Verriegelungselementen entriegelt ist und das zweite Paar von Verriegelungselementen die Arretierfläche des Steckers blockiert.

Aus der DE 31 33 772 C2 ist ein Steckkupplungsventil für zentrale medizinische Gasversorgungsanlagen bekannt, bei dem zwei im Ventilgehäuse auf einer Längsachse verteilt und versetzt angeordnete federbelastete Riegelorgane aus Walzenpaaren oder Kugelkäfigen vorgesehen sind. Um bei einer Abkopplung des zugehörigen Anschlusssteckers vom Ventilgehäuse ein durch Gasdruck erzeugtes unbeabsichtigtes Herausschleudern zu verhindern, soll durch zwei getrennte Schubbewegungen der Schiebehülse sichergestellt werden, dass beim Abkoppeln zunächst der Übergang von der Arbeitsposition in eine Parkposition mit unterbrochener Gaszufuhr und danach durch eine zweite Schubbewegung die endgültige Freigabe des Anschlusssteckers erfolgt. Dies wird dadurch erreicht, dass die im Ventilgehäuse vorgesehenen Führungsnuten für die die Arbeitsposition der Einsteckarmatur sichernden Riegelorgane eine die Schubbewegung der Schiebehülse nach Verschiebung dieser Riegelorgane in ihre Entriegelungsposition begrenzende Anschlagfläche und eine durch eine koaxiale Anschlagfläche begrenzte Ausnehmung aufweisen, die eine Fortsetzung der Schubbewegung der Schiebehülse nach Rückkehr derselben Riegelorgane in ihre Verriegelungsposition erlaubt. Es handelt sich hierbei um eine verhältnismäßig aufwendige Ventilkonstruktion.

Der Erfindung liegt die Aufgabe zugrunde, eine Parkstellung des Steckers in der Entnahmestelle bei unterbrochener Gaszufuhr sicherzustellen, wobei gegenüber der bekannten Vorrichtung eine Teile- und Kostenreduktion erzielt werden soll.

Die Aufgabe wird gelöst, durch ein Steckkupplungsventil gemäß Anspruch 1.

Als besonders vorteilhaft erweist es sich, dass neben einer Teile- und Kostenreduktion auch eine besonders einfache und sichere Handhabung des Kupplungssteckers möglich ist.

Zur Führung des Verriegelungshebels ist dieser im Bereich zwischen der Verriegelungs-Kuppe und dem Anschlag mit wenigstens einem Führungsstift zur Führung in Nuten des Ventilgehäuses versehen; somit ist eine gesicherte Parkposition des Anschlusssteckers vor seiner Entnahme möglich.

In einer bevorzugten Ausführungsform wird aufgrund der zweiten Schubbewegung des Druckrings sowie aufgrund der Entspannung des Federelements der Haken des Verriegelungshebels mit seiner der Arretierfläche des Anschluss-Steckers abgewandten Seite in eine nutförmige Ausnehmung des Mantelsegments von Druckring zwecks Freigabe der Arretierfläche des Anschlusssteckers gebracht, so dass der Anschluss-Stecker entnommen werden kann; die Druckfeder des Druckrings befindet sich nach der Stecker-Entnahme in einer entspannten Position; das als Verriegelungshebel dienende zweite Verriegelungselement besteht aus Kunststoff, vorzugsweise aus Polyamid; auch das Ventilgehäuse und der Druckring bestehen aus Kunststoff.
Im folgenden ist der Gegenstand der Erfindung anhand der Figuren von 1 bis 7 näher erläutert.

Dabei zeigen die
- Figuren 1a bis 1d: das Steckkupplungsventil in seiner Ausgangsstellung ohne eingesetzten Anschluss-Stecker;
- Figuren 2a und 2b: zeigen in einer Draufsicht von der Rückseite aus gesehen (ohne Anschluss-Stecker) und in einer Längsschnittdarstellung das Steckkupplungsventil mit eingesetztem Anschluss-Stecker in Parkstellung,
- Figuren 3a und 3b: zeigen das Steckkupplungsventil in einer Draufsicht der Rückseite ohne Anschluss-Stecker und im Längsschnitt mit eingesetztem Anschlussstecker in der Arbeitsstellung,
- Figuren 4a und 4b: zeigen das Steckkupplungsventil in einer Draufsicht der Rückseite (ohne Anschluss-Stecker) und im Längsschnitt mit Stecker in der Position "Arbeitsstellung entriegeln",
- Figuren 5a und 5b: zeigen das Steckkupplungsventil in einer Draufsicht der Rückseite (ohne Anschluss-Stecker) und im Längsschnitt das Steckkupplungsventil mit dem zu entnehmenden Anschluss-Stecker;
- Figuren 6a und 6b: zeigen das Steckkupplungsventil in einer Draufsicht der Frontseite mit Anschluss-Stecker und einen Längsschnitt entlang der Ebene A-A;
- Figur 7: zeigt den Verriegelungshebel des zweiten Paars von Verriegelungselementen in einer perspektivischen Ansicht.

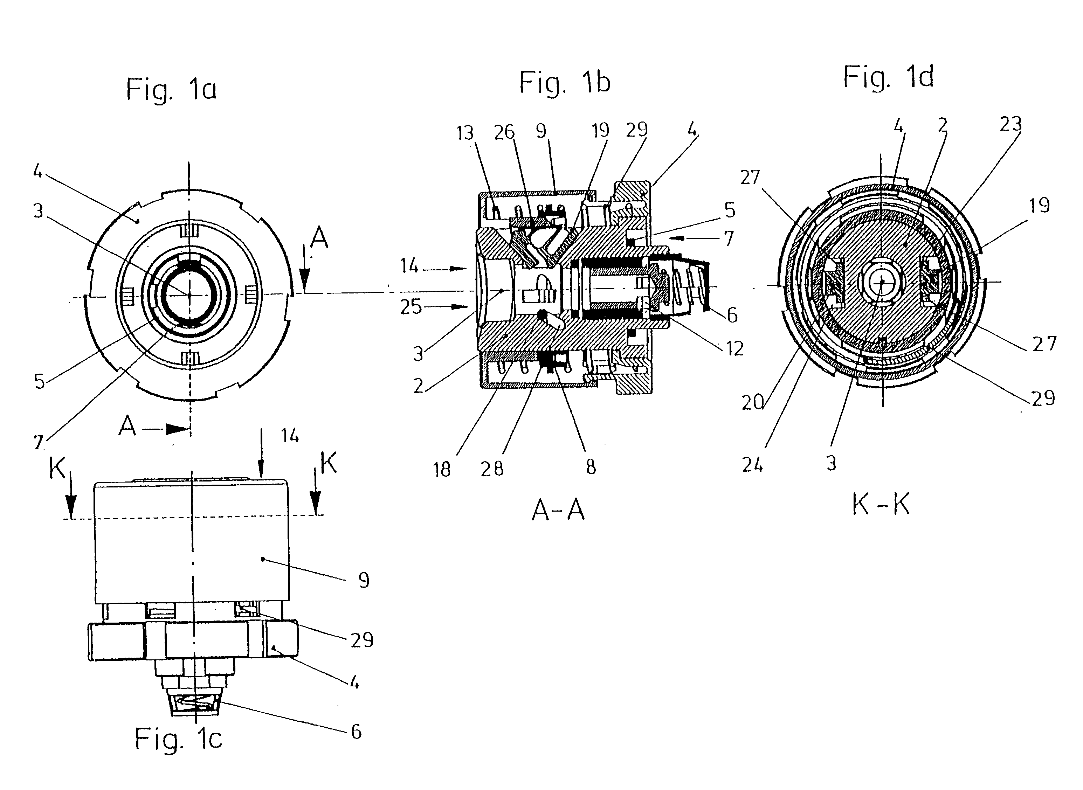

Fig. 1a zeigt die Rückseite des Steckkupplungsventils - vom Benutzer her gesehen -, wobei vom Steckkupplungsventil 1 die Rückseite des Ventilgehäuses 2 erkennbar ist, welches eine Überwurfmutter 4, deren Innengewinde zum Anschluß an die hier nicht dargestellte Gasversorgungsarmatur vorgesehen ist, aufweist. Zur Abdichtung der Gaszuführungsarmatur ist ein O-Ring 5 vorgesehen, der den rohrförmigen Anschlußbereich 7 konzentrisch zur symbolisch dargestellten Längsachse 3 umgibt.

Weiterhin ist in der Längsschnittdarstellung entlang der Ebene A-3-A gemäß Fig. 1b ein als Schiebehülse ausgebildeter Druckring 9 erkennbar, welcher mittels einer konzentrisch zur Längsachse 3 angeordneten Druckfeder 13 in seiner Ausgangsstellung entgegen Pfeilrichtung 14 gepreßt wird. Der Druckring 9 weist in seinem Inneren sich gegenüberliegende zungenartige Mantelsegmente 26 auf, die symmetrisch zur Längsachse 3 angeordnet sind und entlang der Außenoberfläche des Ventilgehäuses 2 gleiten; diese Segmente überlaufen bei Verschiebung des Druckrings 9 sich jeweils diametral gegenüber liegende Ausnehmungen in Ventilgehäuse 2, welche jeweils einen Verriegelungshebel 19, (bzw. 20 in Figur 1b, nicht gezeigt) zur Arretierung des in die Frontöffnung 25 einzuführenden Anschlußsteckers (in seiner Parkposition) aufweisen. Weiterhin ist die als nutförmige Ausnehmung 28 im Ventilgehäuse 2 vorgesehene Führung für das erste Paar von Verriegelungselementen, welche als Verriegelungsstifte 18 ausgebildet sind, erkennbar. Die Verriegelungsstifte 18 werden durch einen mittels Druckfeder 29 belasteten Druckkörper 8 entgegen der Pfeilrichtung 14 gepreßt, so daß sie aufgrund der entgegen der Richtung 14 konisch schräg zulaufenden nutförmigen Ausnehmungen in axialer und radialer Richtung zur Längsache 3 zwecks Arretierung des einzubringenden Anschluß-Steckers hin gepreßt werden.

Entlang der Längsachse 3 ist ein Federgehäuse 6 in das Innengewinde des Anschlußbereiches 7 eingeschraubt. Das Federgehäuse 6 weist in radialer Richtung Gasdurchtrittsöffnungen 12 zur Gasversorgung des einzuführenden Anschlußsteckers auf, der entlang der Längsachse 3 in Richtung des Pfeils 14 eingebracht wird.

Figur 1c stellt die Seitenansicht des Steckventils 1 dar, wobei die Einführungsrichtung für den Anschlußstecker ebenfalls mit Pfeil 14 bezeichnet ist. Weiterhin sind Überwurfmutter 4, Druckfeder 29 (ausschnittsweise) und Druckring 9 sowie Federgehäuse 6 erkennbar.

Figur 1d zeigt einen Querschnitt entsprechend der durch die mittels der Pfeile K in Figur 1c angedeuteten Ebene vorgenommen wurde. In der Schnittdarstellung der Figur 1d ist ein Teil des Ventilgehäuses 2 erkennbar, wobei sich in den Ausnehmungen 27 die Verriegelungshebel 19, 20 befinden, welche jeweils durch Führungsstifte 23, 24 in nutförmigen Ausnehmungen des Ventilgehäuses 2 befinden, die sich in radialer Richtung zur Längsache 3 erstrecken. Die Verriegelungsstifte des ersten Paars von Verriegelungselementen sind hier nicht erkennbar.

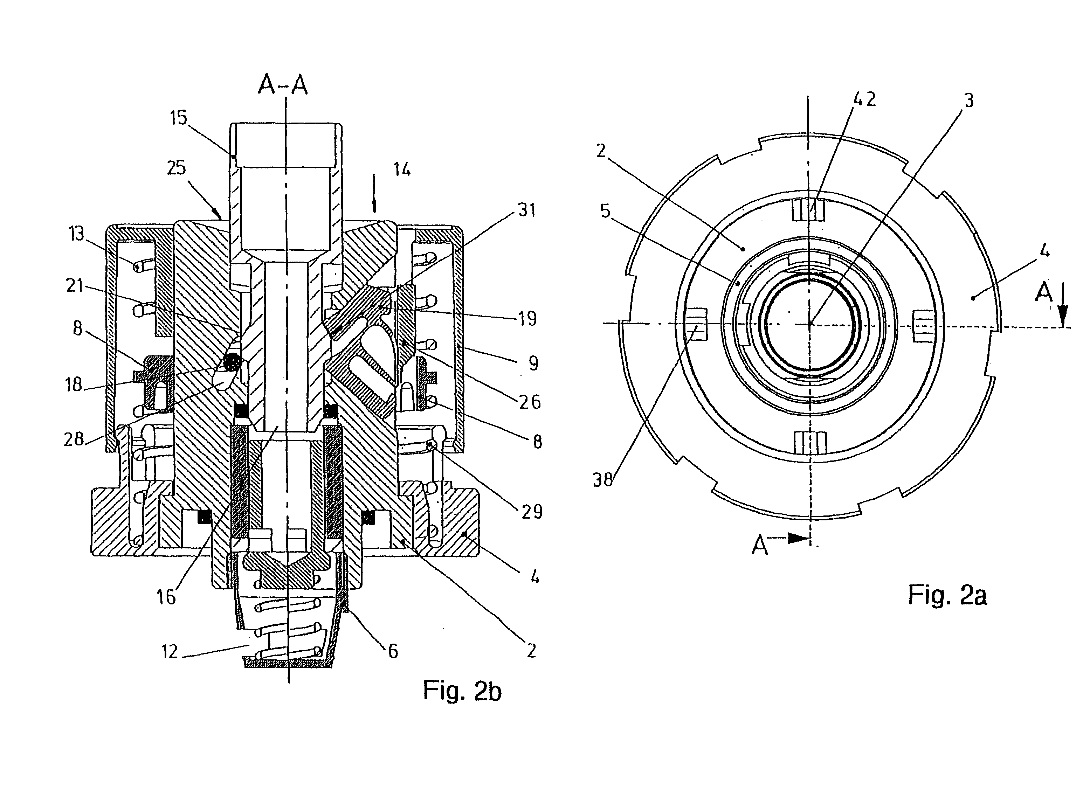

Figur 2a zeigt ebenfalls das Steckkupplungsventil in Rückansicht - vom Benutzer her gesehen - wobei wiederum Überwurfmutter 4, Ventilgehäuse 2 sowie umlaufender O-Ring 5 und Längsachse 3 erkennbar sind; weiterhin sind die als Verdrehschutz für den in das Innengewinde der Überwurfmutter 4 einschraubbaren Gasversorgungsstutzen wirkenden Nocken 42 auf der rückwärtigen Frontseite des Ventilgehäuses 2 erkennbar.

Figur 2b zeigt einen Schnitt entlang der Längsachse A-A der Figur 2a wobei der Teilschnitt A-3 horizontal verläuft, während der Teilschnitt 3-A in vertikaler Richtung verläuft. Somit ist erkennbar, daß der entlang Pfeil 14 in Frontöffnung 25 eingeführte Anschlußstecker 15 mit seiner kegelstumpfartigen Arretierfläche 21 (umlaufender Bund) von der Verriegelungs-Kuppe 31 des Verriegelungshebels 19 (20 ist nicht dargestellt) gegen Herausnahme entgegen Pfeil 14 blokkiert ist. Der Verriegelungshebel selbst befindet sich in vollkommen entspanntem Zustand. Die Unterbrechung der Gaszufuhr erfolgt im Federgehäuse 6. Anhand Figur 2b ist erkennbar, daß die Verriegelungsstifte 18 von dem mit Druckfeder 29 belasteten Druckkörper 8 entgegen der mit Pfeil 14 bezeichneten Richtung in ihre Verriegelungsposition gedrückt werden, wobei sie infolge der nutförmigen Ausnehmung 28 bei Einführen des Anschlußsteckers 15 in Richtung 14 so verschoben werden, daß sie aufgrund der sich schräg in Richtung 14 erweiternden Abstände der nutfömrigen Ausnehmungen 28 im Ventilgehäuse 2 in eine Öffnungsposition für die Einführung bzw. Entnahme des Anschlußsteckers 15 gebracht werden. Druckring 9 ist im Längsschnitt erkennbar zusammen mit der Druckfeder 13. Im übrigen sind die Bezugsziffern denen der Figuren 1a bis 1d angepaßt. Die hülsenförmige Spitze des Anschlußsteckers 15 ist zur Gasaufnahme vorgesehen und mit Ziffer 16 bezeichnet.

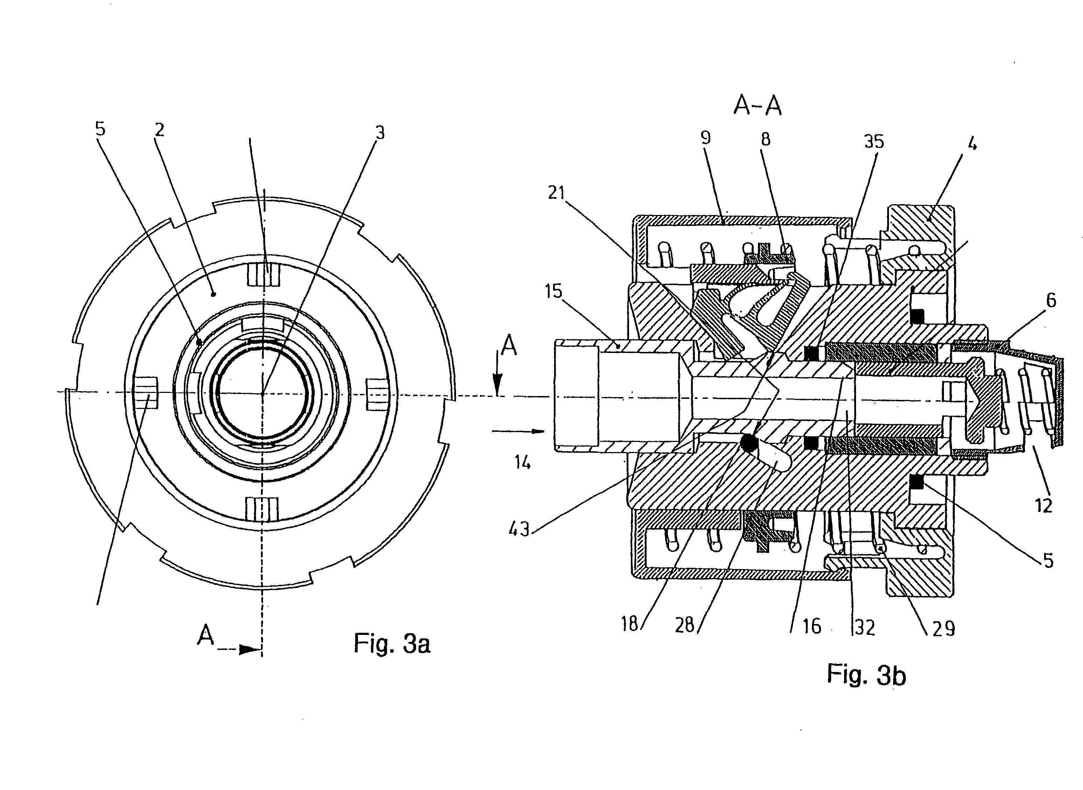

Figur 3a zeigt ähnlich wie Figur 2a die vom Benutzer aus gesehene Rückseitendarstellung, wobei der Gas-Anschlußstecker 15 sich in der Arbeitsstellung befindet wie anhand der Schnittzeichnung A-A (A -3-A) erkennbar ist.

Anhand Figur 3b ist erkennbar, daß Anschlußstecker 15 mit seiner hülsenförmgen Spitze 16 am Ventilstößel 34 der Gaszufuhr anschlägt und diesen in Richtung 14 verschiebt, so daß eine Gaszufuhr über Gasdurchtritt 12 und die Gaszufuhröffnung 32 in den Anschlußstecker 15 möglich ist. Gleichzeitig liegt der mittels Druckkörper 8 und Druckfeder 29 vorgespannte Verriegelungsstift 18 direkt an der Arretierfläche 21 des Anschlußsteckers 15 an, so daß eine Belastung entgegen Richtungspfeil 14 aufgrund der sich konisch verengenden nutförmigen Ausnehmung 28 eine sich selbst verstärkende Blockade von Verriegelungsstift 18 und Arretierungsfläche 21 zur Folge hat. Es handelt sich hierbei um eine blockierende Verriegelung mit sehr extrem hoher Sicherheit.

Die Arretierfläche 21 des umlaufenden Bundes des Anschlußsteckers 15 ist in der Arbeitsstellung gemäß dem oberen Teil der Figur 3b, (d.h. entlang der Schnittebene A-Achse 3 gesehen) ohne Funktion.

Die äußerste Fläche 43 des umlaufenden Bundes des Anschlußsteckers 15 hält den Haken 37 in nach außen verschobener Position, wobei sich der aus einem Kunststoffteil hergestellte Verriegelungshebel 19 bzw. 20 im Gelenk (Filmgelenk) 22 verdreht. Zur besseren Abdichtung des Anschlußsteckers 15 ist dieser im Bereich seiner hülsenförmigen Spitze 16 von einem umlaufenden Dichtring 35 umgeben (O-Ring).

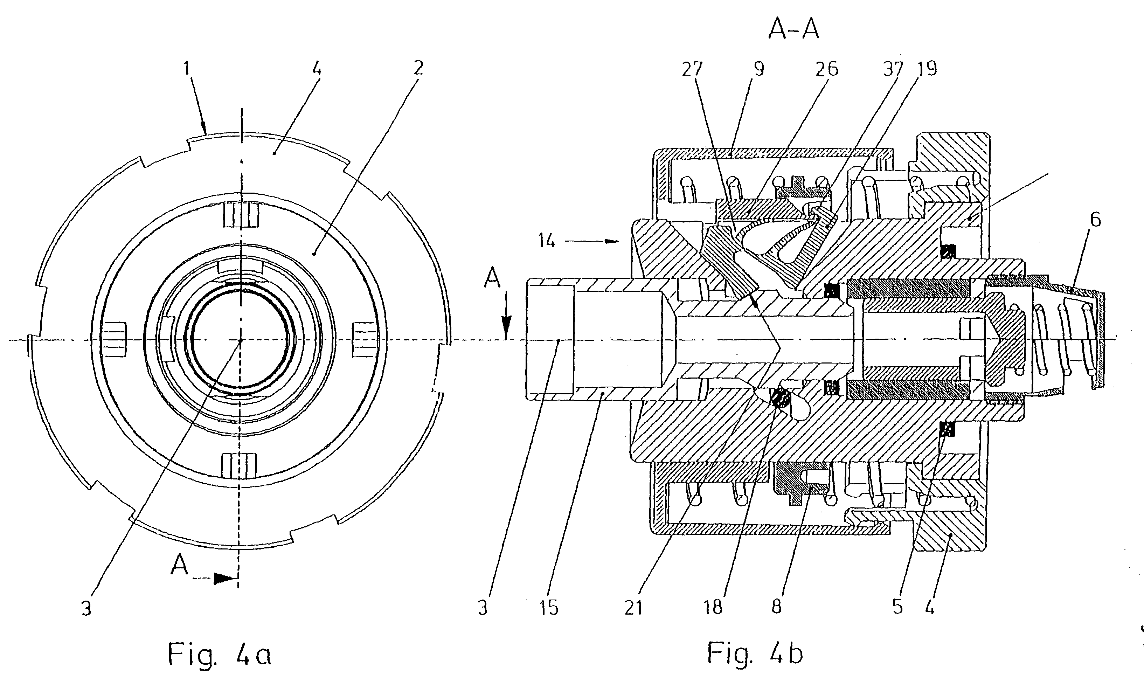

Gemäß Figur 4a zeigt Figur 4b einen Längsschnitt AA durch das Steckkupplungsventil, wobei der obere Abschnitt A-Längsachse 3 in einer horizontalen Ebene verläuft, während der untere Teil der Figur 4b entlang einer vertikalen Ebene (Längsachse 3-A) geschnitten dargestellt ist.

Anhand Figur 4b ist erkennbar, daß nunmehr durch Zusammenpressen des Druckrings 9 in Richtung 14 die zuvor beschriebene Arbeitsstellung entriegelt ist, wobei das erste Paar von Verriegelungselementen 18 nunmehr ohne Funktion ist, während die als Wulst ausgebildete Arretierfläche 21 des Anschlußsteckers 15 nunmehr gegen die Verriegelungskuppe 31 des Verriegelungshebels 19 (bzw. 20, hier nicht dargestellt), stößt. Figur 4b zeigt somit in der Position "Arbeitsstellung entriegeln" das Steckkupplungsventil bei Abbruch der Gasentnahme. Der Benutzer schiebt den Druckring 9 und damit die Verriegelungswalzen 18 in Richtung 14, das bedeutet aus der Sicht des Benutzers "verschieben nach hinten". Die Bewegung des Druckrings 9 wird aufgrund seiner symmetrisch angeordneten zungenartigen Mantelsegmente 26 durch Haken 37 des Verriegelungshebels 19 begrenzt. Aufgrund des Gasdrucks und der Feder am Ventilstößel des Federgehäuses 6 wird der Anschluß-Stecker 15 nach außen getrieben und schlägt an der Verriegelungskuppe 31 des Verriegelungshebels 19 (bzw. 20) an. Der Verriegelungshebel 19 wird durch die Führung 27 als Ausnehmung im Ventilgehäuse 2 nach oben geleitet und in dieser radial gerichteten Bewegung durch Mantelsegment 26 des Druckrings 9 gestoppt.

Der Anschlußstecker 15 kann nicht entnommen werden (entgegen Pfeilrichtung 14), weil der Verriegelungshebel 19 in seiner Verriegelungskuppe 31 den Anschlußstecker 15 an dessen Arretierfläche 21 blockierend berührt und - mit seinem Anschlag 30 durch Mantelsegment 26 blockiert - nicht weiter verschoben werden kann; die weitere Ausziehbewegung des Steckers 15 wird somit unterbunden, so daß eine Gefährdung von Patienten durch abruptes Herausziehen des Anschlußsteckers oder ein Herausfallen von Armaturen verhindert wird.

Nunmehr befindet sich Anschußstecker 15 wiederum in der Parkstellung, wie sie anhand der Figur 2a und 2b bereits erläutert ist. Der Benutzer läßt anschließend den Druckring 9 los, wobei der Druckring 9 durch Federkraft nach vorne gedrückt wird. Der Haken 37 des Verriegelungshebels 19/20 wird dabei von dem Mantelsegment 26 des Druckrings 9 freigegeben und legt sich aufgrund der Entspannung des mit ihm verbundenen elastischen Elements durch Drehung im Gelenk (Filmgelenk) 22 in die Ausnehmung des Ventilgehäuses 2 zurück.

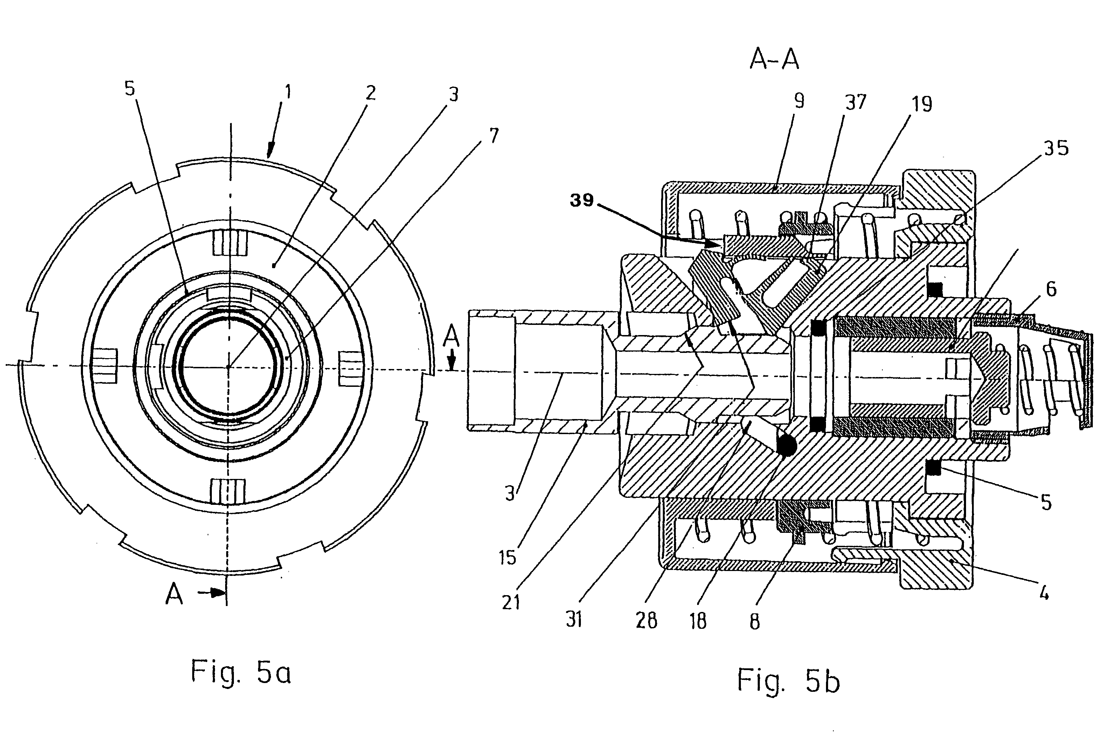

Die Figur 5a und 5b zeigt in einer Darstellung ähnlich den Figuren 3a, 3b oder 4a, 4b einen Schnitt entlang der Achsen A, Längsachse 3-A, wobei erkennbar ist, daß die Position "Steckkupplungsventil Stecker entnehmen" erreicht wird, sobald der Benutzer den Druckring 9 soweit wie möglich erneut nach hinten geschoben hat. Der Haken 37 des Verriegelungshebels 19 (bzw. 20) wird dabei von dem Mantelsegment 26 des Druckrings 9 überlaufen. Die Blockade der Arretierungsfläche 21 durch die Verriegelungskuppe 31 wird aufgehoben, weil der Verriegelungshebel 19 durch die Führung 27 im Ventilgehäuse 2 nach oben geleitet und in dieser Bewegung aufgrund der nutförmigen Ausnehmung 39 im Druckring 9 nicht mehr gestoppt werden kann (im Gegensatz zur Darstellung in Figur 4b). Der Anschlußstecker 15 kann in dieser Stellung entnommen werden, wobei die Verriegelungselemente 18 in die Ausgangsposition und die Verriegelungselemente 19/20 in die Ausgangsstellung durch Federkräfte zurückkehren. Der Druckring 9 kehrt nach Loslassen in die Ausgangsstellung zurück.

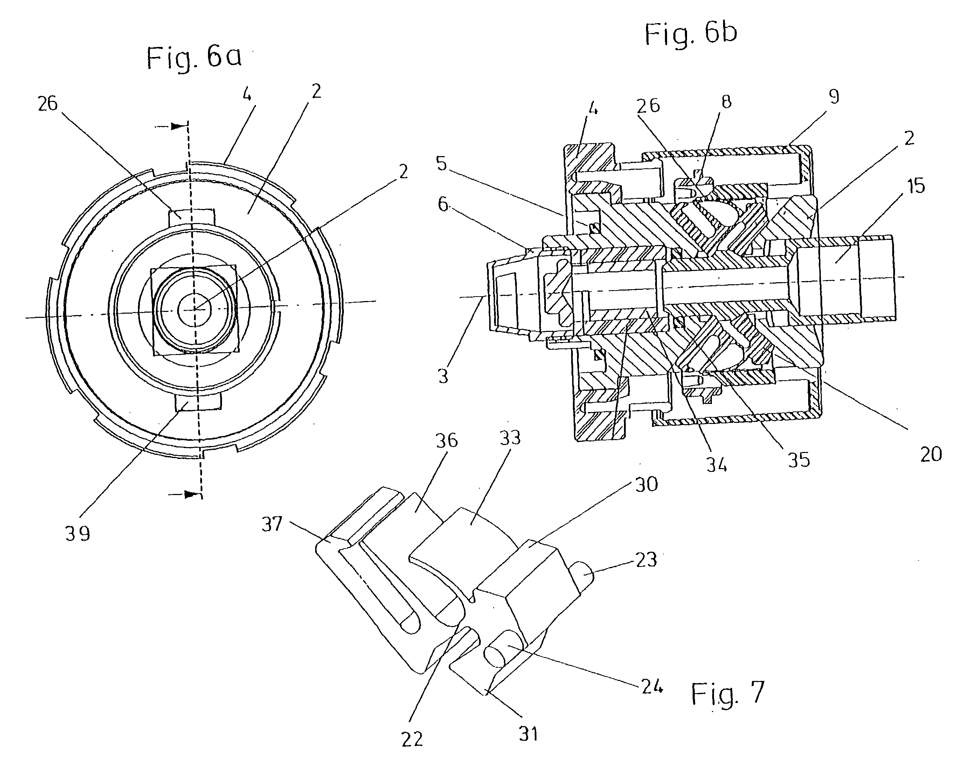

Anhand Fig. 6a ist das Steckkupplungsventil 1 mit seinem Ventilgehäuse in Frontansicht dargestellt. Weiterhin sind die sich symmetrisch gegenüberliegenden nutförmigen Ausnehmungen 39 erkennbar, wie sich auch anhand der entlang der Ebene A-A vorgenommenen Längsschnittdarstellung in Fig. 6b ergibt. Anhand dieser Figur sind beide Verriegelungshebel 19, 20 erkennbar, die mit ihren Verriegelungskuppen 31 noch an der Arretierfläche 21 des Anschlußsteckers 15 ruhen, jedoch aufgrund der Freigabe des Hakens 37 durch die Mantelsegmente 26 bereits zur Entnahme des Anschlußsteckers 15 vorbereitet sind. Im übrigen entspricht diese Längsschnittdarstellung im wesentlichen der Fig. 5b.

Anhand Fig. 7 ist in einer perspektivischen Darstellung ein Verriegelungshebel 19, 20 dargestellt, welcher als zweites Paar von Verriegelungselementen zum Einsatz gelangt. Die zur Blokkade der Arretierfläche des Anschlußsteckers vorgesehene Verriegelungskuppe ist mit Ziffer 31 bezeichnet, während die in einer Ausnehmung des Ventilgehäuses 2 verlaufenden Führungsstifte mit den Ziffern 23, 24 bezeichnet sind. Die Feder ist mit Ziffer 33 bezeichnet. Über Gelenk 22 (Filmgelenk) ist der Haken 37 mit Verriegelungskuppe und Anschlagfläche verbunden, wobei das zum Haken gehörende Federelement 36 in den Verriegelungshebel integriert ist.

Der Verriegelungshebel 19, 20 besteht vorzugsweise aus Kunststoff (Polyamid), wobei die elastische Eigenschaft im Hinblick auf das Gelenk 22 (Filmgelenk) und der Federn 33 und 36 besonders wichtig ist. Verriegelungshebel, die aufgrund ihrer Formgebung steifer als die hier beschriebenen ausfallen, können die hier beschriebenen Funktionen auch erfüllen. Allerdings werden die Betätigungskräfte dann höher sein. Außerdem kann es dann zu unerwünschten Setzerscheinungen kommen.

## Patentansprüche

1. Steckkupplungsventil für zentrale, insbesondere medizinische, Gasversorgungsanlagen mit zwei jeweils paarweise gegenüberliegend auf einer Längsachse (3) eines zugehörigen Ventilgehäuses (2) versetzt angeordneten federbelasteten Verriegelungselementen (18; 19, 20) zur Arretierung eines durch eine Frontöffnung (25) eines federbelasteten Druckrings (9) entlang der Längsachse (3) in seine Arbeitsstellung einsetzbaren Anschluss-Steckers (15) mit hülsenförmiger Spitze (16) zur Gasaufnahme, wobei einerseits das erste Paar von Verriegelungselementen (18) eine Arretierfläche in Form eines die Spitze (16) des Anschluss-Steckers (15) umlaufenden Bundes (21) blockiert, andererseits bei Entnahme des Anschluss-Steckers (15) zwei nacheinander folgende Schubbewegungen des Druckrings (9) mit dazwischen liegender Parkposition vorgesehen sind, wobei in der Parkposition das erste Paar von Verriegelungselementen (18) entriegelt ist und das zweite Paar von Verriegelungselementen (19, 20) den umlaufenden Bund (21) des Anschluss-Steckers (15) blockiert, **dadurch gekennzeichnet, dass** das zweite Verriegelungselement (19, 20) als Verriegelungshebel mit einem Haken (37) ausgebildet ist, wobei der Haken (37) zusammen mit einem Federelement (36) ein elastisches Bauteil bildet, das über ein Gelenk (22) mit einer mit Anschlag versehenen Verriegelungskuppe (31) verbunden ist und wobei der Haken (37) in der Arbeitsstellung radial nach außen gebracht wird durch Kontakt seiner radial nach innen gewandten Seite mit der äußersten Fläche (38) des umlaufenden Bundes (21) des Anschluss-Steckers (15), welcher die erste Schubbewegung des Druckrings (9) anhält durch Anschlag mit den inneren radial sich gegenüberliegenden zungenartigen Mantelsegmenten (26) des Druckrings (9), wobei eine Verriegelungskuppe (31) des Verriegelungshebels den umlaufenden Bund (21) als hintere Arretierfläche des Anschluss-Steckers (15) in der Parkstellung blockiert durch Kontakt seiner radial nach außen gewandten Seite mit der radialen inneren Fläche der Mantelsegmente (26), wobei die Mantelsegmente (26) in einer zweiten Schubbewegung den Haken (37) überlaufen und eine radial nach außen gerichtete Freigabe der radial nach außen gewandten Seite der Verriegelungskuppe (31) erlauben und wobei die Verriegelungshebel jeweils auf der der Verriegelungskuppe (31) abgewandten Seite neben einem Anschlag (30) auch eine Feder (33) zur sicheren Blockade des umlaufenden Bundes (21) als Arretierfläche in der Parkposition des Anschluss-Steckers aufweisen.

2. Steckkupplungsventil nach Anspruch 1, **dadurch gekennzeichnet, dass** zur Führung des Verriegelungshebels dieser im Bereich zwischen Verriegelungskuppe (31) und Anschlag mit wenigstens einem Führungsstift (23, 24) zur Führung in Nuten des Ventilgehäuses (2) versehen ist.

3. Steckkupplungsventil nach Anspruch 1 oder 2, **dadurch gekennzeichnet, dass** aufgrund der zweiten Schubbewegung des Druckrings (9) sowie der Entspannung des verbundenen Federelements (36) der Haken (37) des Verriegelungshebels mit seiner der Arretierfläche (21) des Anschluss-Steckers (15) abgewandten Seite in eine nutförmige Ausnehmung (39) des Mantelsegments von Druckring (9) zwecks Freigabe der Arretierfläche (21) des Anschlusssteckers (15) ragt

4. Steckkupplungsventil nach einem der Ansprüche 1 bis 3, **dadurch gekennzeichnet, dass** die Druckfeder (13) des Druckrings (9) nach der Stecker-Entnahme sich in entspannter Position befindet.

5. Steckkupplungsventil nach einem der Ansprüche 1 bis 4, **dadurch gekennzeichnet, dass** das als Verriegelungshebel dienende zweite Verriegelungselement aus Kunststoff, vorzugsweise aus Polyamid, besteht.

6. Steckkupplungsventil nach einem der Ansprüche 1 bis 5, **dadurch gekennzeichnet, dass** Ventilgehäuse (2) und Druckring (9) aus PBT (Polybutylenterephthalat) bestehen.

## Claims

1. Plug-in coupling valve for centralized, especially medical, gas supply systems, with two offset pairs of spring-loaded locking elements (18; 19, 20), the elements of each pair being arranged opposite each other on a longitudinal axis (3) of an associated valve casing (2), for locking a plug connector (15) with a sleeve-like tip (16) for receiving gas, insertable along the longitudinal axis (3) into its working position through a front opening (25) of a spring-loaded thrust collar (9), wherein, on the one hand, the first pair of locking elements (18) jam a locking face in the form of a shoulder (21) surrounding the tip (16) of the plug connector (15), and on the other hand, upon withdrawal of the plug connector (15) provision is made for two successive thrust movements of the thrust collar (9) with an intermediate parking position, such that in the parking position the first pair of locking elements (18) are unlocked and the second pair of locking elements (19, 20) jam the circumferential shoulder (21) of the plug connector (15), **characterized in that** the second locking element (19, 20) is configured as a locking lever with a hook (37) forming in conjunction with a springy element (36) an elastic component connected by a hinge (22) to a locking lug (31) provided with a stop, the hook (37) being displaced radially outwards in the working position through contact of its radially inwards facing side with the outermost face (38) of the circumferential shoulder (21) of the plug connector (15), which checks the first thrust movement of the thrust collar (9) by abutment against the inner radially opposite tongue-like shell segments (26) of the thrust collar (9), a locking lug (31) of the locking lever jamming the circumferential shoulder (21) in the parking position as a rear locking face of the plug connector (15) through contact of its radially outwards facing side with the radial inner face of the shell segments (26), the shell segments (26) overriding the hook (37) in a second thrust movement and allowing a radially outwards directed release of the radially outwards facing side of the locking lug (31), and each locking lever having on the side facing away from the locking lug (31), in addition to a stop (30), a spring (33) for positive jamming of the circumferential shoulder (21) as locking face in the parking position of the plug connector.

2. Plug-in coupling valve according to Claim 1, **characterized in that** the locking lever is provided in the region between locking lug (31) and stop with at least one guide pin (23, 24) to guide the locking lever in grooves in the valve casing (2).

3. Plug-in coupling valve according to Claim 1 or 2, **characterized in that** as a result of the second thrust movement of the thrust collar (9) and relaxation of the connected spring element (36) the hook (37) of the locking lever projects, by its side facing away from the locking face (21) of the plug connector (15), into a slot-like recess (39) in the shell segment of the thrust collar (9) in order to release the locking face (21) of the plug connector (15).

4. Plug-in coupling valve according to any one of Claims 1 to 3, **characterized in that** the compression spring (13) of the thrust collar (9) is in its relaxed position after the plug is withdrawn.

5. Plug-in coupling valve according to any one of Claims 1 to 4, **characterized in that** the second locking element serving as locking lever is made of plastic material, preferably polyamide.

6. Plug-in coupling valve according to any one of Claims 1 to 5, **characterized in that** valve casing (2) and thrust collar (9) are made of PBT (polybutyleneterephthalate).

## Revendications

1. Raccord enfichable à soupape pour systèmes d'alimentation en gaz, en particulier médicaux, comprenant deux éléments de verrouillage (18 ; 19, 20) chargés par ressort respectivement disposés en déport par paires et en vis-à-vis sur un axe longitudinal (3) d'une cage de soupape (2) correspondante pour le blocage d'une fiche de raccordement (15), munie d'une pointe (16) en forme de manchon pour recevoir du gaz et qui peut être mise en place dans sa position de travail, le long de l'axe longitudinal (3), au travers d'une ouverture frontale (25) d'une bague de serrage (9) chargée par ressort, la première paire d'éléments de verrouillage (18) bloquant une surface d'arrêt en forme d'un collet (21) périphérique à la pointe (16) de la fiche de raccordement (15), d'un côté, deux mouvements de poussée successifs de la bague de serrage (9), avec une position d'attente intermédiaire, étant prévus lors du retrait de la fiche de raccordement (15), d'un autre côté, la première paire d'éléments de verrouillage (18) étant déverrouillée et la seconde paire d'éléments de verrouillage (19, 20) bloquant le collet périphérique (21) de la fiche de raccordement (15) dans la position d'attente, **caractérisé en ce que** le second élément de verrouillage (19, 20) est réalisé sous forme de levier de verrouillage avec un crochet (37), le crochet (37) formant avec un élément à ressort (36) un composant élastique assemblé par l'intermédiaire d'une articulation (22) avec une calotte de verrouillage (31) munie d'une butée, et le crochet (37) étant amené radialement vers l'extérieur dans la position de travail par contact de son côté, tourné radialement vers l'intérieur, avec la surface extérieure (38) du collet périphérique (21) de la fiche de raccordement (15), qui retient le premier mouvement de poussée de la bague de serrage (9) par butée avec les segments d'enveloppe intérieurs (26) en forme de languettes de la bague (9) en vis-à-vis dans la direction radiale, une calotte de verrouillage (31) du levier de verrouillage bloquant dans la position d'attente le collet périphérique (21), en tant que surface d'arrêt arrière de la fiche de raccordement (15), par contact de son côté, tourné radialement vers l'extérieur, avec la surface radiale intérieure des segments d'enveloppe (26), les segments d'enveloppe (26) dépassant le crochet (37) dans un second mouvement de poussée et permettant un dégagement, dirigé radialement vers l'extérieur, du côté de la calotte de verrouillage (31) tourné radialement vers l'extérieur, et chaque levier de verrouillage présentant sur le côté opposé à la calotte (31), outre une butée (30), un ressort (33) pour le blocage sûr du collet périphérique (21) en tant que surface d'arrêt dans la position d'attente de la fiche de raccordement.

2. Raccord enfichable à soupape suivant la revendication 1, **caractérisé en ce que** le levier de verrouillage est muni pour son guidage d'au moins une goupille de guidage (23, 24) dans la zone comprise entre la calotte de verrouillage (31) et la butée, pour le guidage dans des rainures de la cage de soupape (2).

3. Raccord enfichable à soupape suivant la revendication 1 ou 2, **caractérisé en ce que**, du fait du second mouvement de poussée de la bague de serrage (9) ainsi que de la détente de l'élément à ressort (36) assemblé, le crochet (37) du levier de verrouillage pénètre par son côté opposé à la surface d'arrêt (21) de la fiche de raccordement (15) dans un creux (39) en forme de rainure du segment d'enveloppe de la bague de serrage (9) pour dégager la surface d'arrêt (21) de la fiche de raccordement (15).

4. Raccord enfichable à soupape suivant l'une des revendications 1 à 3, **caractérisé en ce que** le ressort de compression (13) de la bague de serrage (9) se situe dans la position de détente après le retrait de la fiche.

5. Raccord enfichable à soupape suivant l'une des revendications 1 à 4, **caractérisé en ce que** le second élément de verrouillage, servant de levier de verrouillage, se compose de matière plastique, de préférence de polyamide.

6. Raccord enfichable à soupape suivant l'une des revendications 1 à 5, **caractérisé en ce que** la cage de soupape (2) et la bague de serrage (9) se composent de PBT (polybutylènetéraphtalate).
